# EUROPÄISCHE PATENTSCHRIFT

(11) **EP 4 056 915 B1**
(45) Veröffentlichungstag und Bekanntmachung des Hinweises auf die Patenterteilung: **24.05.2023**
(21) Anmeldenummer: 21161995.2
(22) Anmeldetag: 11.03.2021
(51) Int. Cl.: F24F 8/22, A61L 9/20, F24F 11/38, F24F 11/32

(54) **RAUMLUFTREINIGER AUFWEISEND EINEN OZONGENERATOR MIT EINER UV-LAMPE UND VERFAHREN ZUM BETREIBEN EINES RAUMLUFTREINIGERS**
ROOM AIR PURIFIER COMPRISING AN OZONE GENERATOR WITH A UV LAMP AND METHOD FOR OPERATING A ROOM AIR PURIFIER
DISPOSITIF DE NETTOYAGE DE L'AIR AMBIANT COMPRENANT UN GÉNÉRATEUR D'OZONE DOTÉ D'UNE LAMPE UV ET PROCÉDÉ DE FONCTIONNEMENT D'UN DISPOSITIF DE NETTOYAGE DE L'AIR AMBIANT

(43) Veröffentlichungstag der Anmeldung: 14.09.2022
(73) Patentinhaber: Brunauer, Thomas Sebastian, 5020 Salzburg (AT)
(72) Erfinder: STEINBACHER, Andreas, 5082 Grödig (AT); SEELENBACHER, Klaus, 5400 Hallein/Taxach (AT); AUER, Johannes, 5020 Salzburg (AT)
(74) Vertreter: Karakatsanis, Georgios

(56) Entgegenhaltungen:
- WO-A2-2016/081959
- US-A- 5 612 001
- US-A1- 2005 000 365
- US-A1- 2013 239 803

## Beschreibung

Die vorliegende Erfindung bezieht sich auf einen Raumluftreiniger aufweisend einen Ozongenerator mit einer UV-Lampe gemäß dem Oberbegriff des Patentanspruchs 1. Ferner bezieht sich die Erfindung auf ein Verfahren zum Betreiben des erfindungsgemäßen Raumluftreinigers.

Aus dem Stand der Technik sind Raumluftreiniger bekannt, welche zumindest einen Ozongenerator aufweisen, der in einem Gehäuse mit einer Lufteintritts- und einer Luftaustrittsöffnung angeordnet ist. Hierbei ist ein Lüfter vorgesehen, welcher an der Lufteintrittsöffnung angeordnet ist und Luft in das Innere des Gehäuses ansaugt. Die Luft passiert den Ozongenerator, wobei sich das Ozon mit Geruchsmolekülen der Luft verbindet und Gerüche, Keime, Viren und Bakterien eliminiert. Die im Gerät gereinigte Luft wird anschließend in den Raum rückgeführt.

Ferner sind derartig ausgeführte Raumluftreiniger bekannt, bei denen Ozon durch einen Ozongenerator erzeugt und mittels des an der Lufteintrittsöffnung angeordneten Lüfters aus dem Gerät in den Raum geblasen wird.

Aus der WO 2016/081959 A2 ist ein Raumluftreiniger gemäß dem Oberbegriff von Anspruch 1 bekannt. Die WO 2016/081959 A2 offenbart einen Raumluftreiniger umfassend ein Gehäuse, in dem entlang einer Längsachse des Gehäuses oder achsparallel dazu ein Ozongenerator mit einer UV-Lampe angeordnet ist, wobei axial betrachtet an einer Stirnseite des Gehäuses eine Lüftereinheit, deren Drehzahl einstellbar ist, angeordnet ist. Bei dem bekannten Raumluftreiniger ist die elektrische Leistung und somit die UV-Licht -Strahlungsleistung einstellbar; ferner wird die Lebensdauer der UV-Lampe anhand der Anzahl der Betriebsstunden geschätzt basierend auf die Betriebsstunden der Charge der UV-Lampen bevor ein vorgegebener Prozentsatz der Lampen ausgefallen ist.

Ferner geht aus der US 5 612 001 A eine Luftreinigungsvorrichtung mit einer inneren ellipsoiden Kammer, die UV-Lampen entlang der Hauptachse des Ellipsoids enthält, wobei jedes der Enden des Ellipsoids entlang der Hauptachse des Ellipsoids eine Öffnung aufweist, die den Eintritt und Austritt von Luft aus der Umgebung ermöglicht. Hierbei sind an der Basis jeder UV-Lampe Sensoren angebracht, wobei ein Lampenausfall erkannt wird, wenn die Leistung einer UV-Lampe um mehr als 20 % unter den Normalwert fällt. Ferner sind Sensoren vorgesehen, welche erfassen, ob die Lüftereinheiten der Luftreinigungsvorrichtung die vorgesehene Drehzahl aufweisen.

Der vorliegenden Erfindung liegt die Aufgabe zugrunde, einen Raumluftreiniger aufweisend einen Ozongenerator mit zumindest einer UV-Lampe anzugeben. Des Weiteren soll ein Verfahren zum Betreiben eines Raumluftreinigers, insbesondere eines erfindungsgemäßen Raumluftreinigers angegeben werden.

Diese Aufgabe wird für einen Raumluftreiniger durch die Merkmale des Patentanspruchs 1 gelöst. Ein Verfahren zum Betreiben eines Raumluftreinigers ist Gegenstand des Patentanspruchs 11. Weitere erfindungsgemäße Ausgestaltungen und Vorteile gehen aus den entsprechenden Unteransprüchen hervor.

Demnach wird ein Raumluftreiniger, im Folgenden auch Gerät genannt, vorgeschlagen, umfassend ein Gehäuse, in dem entlang einer Längsachse des Gehäuses, welche der Richtung seiner größten Ausdehnung entspricht oder achsparallel dazu ein Ozongenerator mit einer UV-Lampe angeordnet ist, wobei axial betrachtet an einer Stirnseite des Gehäuses eine Lüftereinheit angeordnet ist, welche eine Strömung von oder zu einer axial betrachtet gegenüberliegenden Stirnseite des Gehäuses verursacht, wobei an beiden Stirnseiten des Gehäuses eine Öffnung vorgesehen ist, wobei in Abhängigkeit der durch die Lüftereinheit verursachten Strömungsrichtung der Strömung eine Öffnung als Lufteintrittsöffnung und die andere Öffnung als Luftaustrittsöffnung ausgeführt ist, über die in das Geräteinnere angesaugte und mit Ozon angereicherte durchströmende Luft ausgestoßen wird.

Gemäß der Erfindung weist der Raumluftreiniger eine einstellbare Treiberschaltung auf, welche nach einem Signal eines Mikrocontrollers die UV-Lampe zündet und derart betreibt, dass die elektrische Leistung und somit die UV-Licht Strahlungsleistung einstellbar ist, wobei ein Übertrager vorgesehen ist, über den der Status der UV-Lampe (gezündet, nicht gezündet, Zeit bis Zündung) ausgekoppelt wird, wobei der Status der UV-Lampe über einen Optokoppler des Raumluftreinigers erfasst wird und an den Mikrocontroller zur Auswertung zum Zweck einer Lampenausfall - Früherkennung übermittelt wird.

Auf diese Weise kann erkannt werden ob die UV-Lampe ihr Lebensdauerende erreicht hat, bevor es zu einem Lampenausfall kommt; ferner kann erkannt werden, ob die UV-Lampe defekt ist, nicht mehr, oder erst nach mehreren Zündversuchen zündet.

Vorzugsweise ist die Treiberschaltung als Resonanzkonverter ausgeführt, wobei die Leistungseinstellung der UV-Lampe durch die Betriebsfrequenz und die Eingangsspannung verändert werden kann.

Die Lüftereinheit ist mittels des Mikrocontrollers steuerbar und derart ausgeführt, dass die Drehzahl und somit der Luftdurchsatz bzw. die Auswurfstärke der Luft einstellbar ist, wobei in die Lüftereinheit ein mit dem Mikrocontroller verbundener Sensor integriert ist, welcher die Drehzahl der Lüftereinheit erfasst und an den Mikrocontroller zur Auswertung zum Zweck einer Lüftereinheitausfall - Früherkennung weiterleitet. Führt ein Schaden zu niedrigerer Drehzahl als nominal erwartet, was beispielsweise anhand einer Kennlinie erkannt wird, kann damit ein bevorstehender Lüfterausfall erkannt und per Netzwerk und WLAN, IOT (Internet of things) oder einer Anzeige gemeldet werden. Durch Auswertung der Drehzahl der Lüftereinheit kann erkannt werden, ob die Lüftereinheit ihr Lebensdauerende erreicht hat, was beispielsweise bei einer durch defekte Lager ausgelösten Drehzahlreduzierung der Fall ist. Ferner kann erkannt werden, ob die Lüftereinheit stillsteht.

Bei Erkennung, dass die UV-Lampe oder die Lüftereinheit defekt sind oder das Ende ihrer Lebensdauer erreicht haben, werden über ein Modul zur drahtlosen Kommunikation und ein Netzwerk (z.B. WLAN und Internet) entsprechende Daten an einen Server übermittelt, wobei anschließend der Benutzer informiert, ein Servicetermin vereinbart oder ein Ersatzteil angeboten werden kann.

Gemäß einer Ausgestaltung der Erfindung kann die UV-Lampe radial betrachtet von einem Luftleitbauteil mit einem vorgegebenen Abstand in einem vorgegebenen Winkelbereich umgeben sein, wobei zumindest die der UV-Lampe zugewandte Seite des Luftleitbauteils für die von der UV-Lampe emittierte Strahlung reflektierend ausgeführt sein kann. Dadurch wird der Bestrahlungsraum innerhalb des Raumluftreinigers und somit der Ozonausstoß verringert, da die Möglichkeit einer Interaktion der UV - Strahlung mit O2 eingeschränkt wird.

Gemäß einer Ausgestaltung der Erfindung kann der Raumluftreiniger in Strömungsrichtung der Luft nach der UV-Lampe zumindest ein fest oder mittels eines Aktuators beweglich angeordnetes Stauelement vorgegebener Größe aufweisen, welches beispielsweise als Platte oder Lochplatte aus Kunststoff oder Metall ausgeführt sein kann, durch das ein Rückstau der Luft erzielt wird, was zu einer stärkeren Sättigung der Luft im Bestrahlungsraum führt. Vorzugsweise sind die Stauelemente senkrecht zur Luftstromrichtung angeordnet bzw. für den Fall von beweglich angeordneten Stauelementen anordenbar. Gemäß einer Weiterbildung kann ein mittels eines Aktuators beweglich angeordnetes Stauelement nahe der Auslassöffnung vorgesehen sein, wodurch eine Veränderung des Ausstoßes, der Sättigung der Luft und des Volumenstroms erreicht wird.

Gemäß einer weiteren Ausgestaltung der Erfindung weist der Raumluftreiniger ein mittels eines Aktuators axial beweglich angeordnetes Stauelement vorgegebener Größe, welches in einem vorgegebenen Abstand zur UV-Lampe anordenbar ist, derart, dass es einen Teil der UV-Lampe abdeckt. Auf diese Weise wird der Bestrahlungsraum innerhalb des Raumluftreinigers und somit der Ozonausstoß definiert verringert, da die Möglichkeit einer Interaktion der UV - Strahlung mit O2 eingeschränkt wird.

Gemäß einer Ausgestaltung der Erfindung weist die Stirnseite des Gehäuses, der die Luftaustrittsöffnung zugeordnet ist, einen geringeren Durchmesser auf als das Gehäuse und ist axial betrachtet um ein vorgegebenes Maß nach außen verschoben angeordnet.

Im Rahmen einer Weiterbildung dieser Ausgestaltung ist vorgesehen, dass die Stirnseite des Gehäuses, der die Luftaustrittsöffnung zugeordnet ist und die einen geringeren Durchmesser als das Gehäuse aufweist mittels eines Aktuators zwischen einer ersten und einer zweiten Position axial stufenlos verschiebbar ausgeführt ist. In der ersten Position, bei der die gedachte Radiale Fortsetzung der Stirnseite am Rand des Gehäuses anliegt, wird die mit Ozon angereicherte Luft geradlinig und weit ausgestoßen, wobei in der zweiten Position, bei der die Stirnseite um einen Maximalwert nach außen verschoben angeordnet die mit Ozon angereicherte Luft in den Raum in einem Winkelbereich zwischen 0 und 90° bezogen auf die Längsachse gleichmäßig konzentriert ausgestoßen wird. Durch eine entsprechende Ansteuerung des Aktuators kann in vorteilhafter Weise ein zwischen der ersten und der zweiten Position oszillierender Luftausstoß realisiert werden.

Der Ozonanteil im Gasgemisch ergibt sich aus der eingestellten Leistung der UV-Lampe, der Drehzahl des Lüfters und falls vorhanden der Einschränkung des Luftstroms mittels Stauelementen, die dem Luftstrom einen Widerstand entgegensetzen oder ihn einschnüren.

Gemäß einer weiteren Ausgestaltung der Erfindung weist das Gehäuse einen ersten Abschnitt auf, welcher dem beschriebenen Gehäuse entspricht, mit dem Unterschied, dass die UV-Lampe nicht in diesem Abschnitt, sondern in einem zweiten Gehäuseabschnitt angeordnet ist, der vom ersten Gehäuseabschnitt durch eine für die UV-A und UV-B Strahlung der UV-Lampe über einen definierten Bereich durchlässigen Wandabschnitt aus beispielsweise Glas oder PMMA getrennt ist und durch eine in der Nähe der Auslassöffnung des ersten Gehäuseabschnitts vorgesehene variable Öffnung, welche als Einlassöffnung zum ersten Gehäuseabschnitt dient, mit dem ersten Gehäuseabschnitt strömungstechnisch verbunden ist. Die Einlassöffnung zum ersten Gehäuseabschnitt kann auch nicht variabel ausgeführt sein.

Die variable Öffnung kann als mittels eines Aktuators verstellbare Blende ausgeführt sein. Alternativ kann die variable Öffnung als elektronisch regelbare Düse ausgeführt sein.

Im zweiten Gehäuseabschnitt ist eine weitere Lüftereinheit vorgesehen, die über einen Anschluss für Um-, Außenluft oder gereinigte Raumluft einen an der UV-Lampe vorbeiströmenden Luftstrom oder in Abhängigkeit der Anordnung der UV-Lampe einen die UV-Lampe umströmenden Luftstrom in Richtung der Einlassöffnung zum ersten Gehäuseabschnitt generiert.

Der erste Gehäuseabschnitt dient als Reinigungskammer, in der die in das Geräteinnere angesaugte, z.B. durch Fett, Dreck, Staub verschmutzte Luft mit UV-A und UV-B Licht behandelt und gereinigt wird, wobei der zweite Gebäudeabschnitt als Ozon-Generierungskammer dient, wobei die mit Ozon angereicherte Luft der in der Reinigungskammer gereinigten Luft in Strömungsrichtung vor der Luftauslassöffnung des ersten Gehäuseabschnitts direkt oder in einer mit der Luftauslassöffnung des ersten Gehäuseabschnitts strömungstechnisch verbundenen Mischkammer regelbar beigemischt wird. Gemäß einer vorteilhaften Ausgestaltung können in der Reinigungskammer zumindest eine HEPA Filterstufe und ein Fettfilter aus Metall vorgesehen sein.

Durch die zwei- und drei-Kammer Ausgestaltung werden in vorteilhafter Weise ein separat regelbarer UV-Output zum Zweck der Luftreinigung sowie eine separat regelbare Ozon-Generierung und Ozon Beimischung erzielt. Des Weiteren kann die Reinigungskammer auf einfache Weise gefahrlos gereinigt werden, wobei die Elektronik und die UV-Lampe weitgehend vor Verschmutzung geschützt sind. Ein weiterer Vorteil besteht darin, dass der Raumluftreiniger ohne Ozon-Generierung betrieben werden kann.

Gemäß weiterer Ausgestaltungen der Erfindung kann der Raumluftreiniger Sensoren aufweisen oder mit Sensoren über einen geeigneten Standard zur drahtlosen Kommunikation, beispielsweise über WLAN, WLAN und Internet, Bluetooth etc. verbindbar sein, wobei die Sensoren vom Raumluftreiniger periodisch abgefragt oder periodisch Sensordaten an den Raumluftreiniger senden, der anhand der Sensordaten gesteuert werden kann.

Beispielsweise kann ein Sensor als Anwesenheitssensor oder Mikrofon und ein weiterer Sensor als Sensor zur Erfassung der Ozonkonzentration ausgeführt sein. Hierbei kann der Sensor zur Erfassung der Ozonkonzentration verwendet werden, um die Ozonkonzentration im Raum nicht über einen Schwellenwert steigen zu lassen; der Raumluftreiniger wird anhand der erfassten Ozonkonzentration gesteuert. In Verbindung mit den Signalen eines Anwesenheitssensors kann der Raumluftreiniger z.B. derart betrieben werden, dass sich das Gerät selbständig aktiviert, wenn keine Person im Raum ist, wobei die durch den Sensor zur Erfassung der Ozonkonzentration erfasste Ozonkonzentration im Raum aktiv hochgeregelt wird, wenn keine Person anwesend ist und zurückgeregelt wird, wenn die Anwesenheit von Personen erfasst wird.

Der Sensor zur Erfassung der Ozonkonzentration kann auch verwendet werden, um den Zustand der UV-Lampe zu überprüfen. Hierbei wird die Ozonkonzentration nach dem Einschalten des Gerätes kontinuierlich erfasst, wobei, wenn diese nicht wie erwartet in Abhängigkeit der Raumgröße und des Abstandes des Sensors zur Erfassung der Ozonkonzentration zum Gerät steigt, ein Defekt der UV-Lampe erkannt wird.

Ferner kann ein Sensor als Luftqualitätssensor ausgeführt sein, anhand dessen Signale das Gerät aktiviert wird, bis die Luftqualität einen voreingestellten Schwellenwert überschreitet oder für eine einstellbare Dauer oder Maximaldauer.

Gemäß der Erfindung können mehrere Raumluftreiniger zentral gesteuert werden; beispielsweise können in einem Hotel per zentraler Steuerung in einem Netzwerk über WLAN sämtliche Raumluftreiniger gesteuert werden.

Erfindungsgemäße Raumluftreiniger können einer Gruppe (synch-Verbund) zugeordnet werden, wobei beliebige Geräte zu einer Gruppe zugeordnet oder herausgenommen werden können. Hierbei reagieren sämtliche derselben Gruppe zugeordneten Geräte synchronisiert oder definiert zu Bedienereingaben an einem der dieser Gruppe zugeordneten Gerät, wobei die Art der Bedienereingabe lokal, über ein Netzwerk und ein Modul zur drahtlosen Kommunikation oder über eine Fernbedienung erfolgen kann.

Die synchrone Steuerung der Geräte einer Gruppe kann dadurch erfolgen, dass in den Geräten jeder Gruppe eine eindeutige Kennung (z.B. Adresse oder Seriennummer) sämtlicher Geräte der Gruppe gespeichert ist, so dass, wenn ein Gerät dieser Gruppe eine Bedienereingabe empfängt, diese ausführt und adressiert an alle Geräte in derselben Gruppe weiter sendet.

Alternativ kann ein Gerät einer Gruppe nach Erhalt einer Bedienereingabe diese ausführen und einen Broadcast - Befehl an alle Geräte in allen Gruppen senden, welcher an die Geräte der Gruppe, der das Gerät zugeordnet ist, adressiert ist. Da in jedem Gerät Informationen über die Gruppenzugehörigkeit gespeichert sind, wird die Bedienereingabe nur von den Geräten der Gruppe, der das broadcastende Gerät zugeordnet ist, ausgeführt.

Gemäß einer weiteren Ausgestaltung können die Geräte einer Gruppe gleichzeitig über ein Netzwerk und die Module zur drahtlosen Kommunikation der Geräte gesteuert werden.

Ferner können Geräte einer Gruppe, die sich am selben Ort befinden, durch eines der Geräte anhand von Signalen eines einem Gerät zugeordneten Luftqualitätssensors gesteuert werden. Wenn sich beispielsweise Geräte in der Lobby eines Hotels befinden und mittels des Luftqualitätssensors eines dieser Geräte detektiert wird, dass die Luftqualität einen Schwellwert unterschritten hat, wird das Gerät, dem der Luftqualitätssensor zugeordnet ist, aktiviert, wobei die weiteren Geräte der Gruppe sofort oder nach Ablauf eines vorgegebenen, einstellbaren Timers, wenn bis zum Ablauf des Timers die Luftqualität nicht in einem vorgegebenen Maße verbessert ist, aktiviert werden und wobei anschließend alle Geräte deaktiviert werden, wenn die Luftqualität den Schwellenwert nicht mehr unterschreitet. Alternativ werden anschließend alle Geräte deaktiviert, wenn die Luftqualität einen weiteren Schwellenwert überschreitet, der höher als der Schwellenwert ist, welcher die Aktivierung verursacht hat.

Die Erfindung wird im Folgenden anhand der beigefügten Figuren beispielhaft näher erläutert. Der allgemeine Aufbau und die Funktionsweise von Raumluftreinigern umfassend einen Ozongenerator mit einer UV-Lampe sind dem Fachmann bestens bekannt, so dass im Folgenden die Merkmale detailliert beschrieben werden, die für die vorliegende Erfindung von Bedeutung sind. Es zeigen:
Figur 1: Eine schematische Ansicht zur Veranschaulichung der einstellbaren Treiberschaltung, welche die UV-Lampe des Ozongenerators zündet und betreibt;
Figur 2: Ein Blockschaltbild zur Veranschaulichung der einstellbaren Treiberschaltung, deren Spannungsversorgung und der Verbindung der Treiberschaltung mit dem Mikrocontroller zur Auswertung zum Zweck einer Lampenausfall - Früherkennung;
Figur 3: Eine schematische, vereinfachte Ansicht basierend auf Figur 2, zur Veranschaulichung Messung der Dauer bis zur Zündung der UV-Lampe;
Figur 4: Eine schematische Ansicht zur Veranschaulichung der Steuerung der Lüftereinheit durch den Mikrocontroller und der Lüfterausfall-Früherkennung ;
Figur 5: Eine schematische Schnittansicht eines Teiles eines erfindungsgemäßen Raumluftreinigers gemäß einer Ausgestaltung der Erfindung;
Figur 6: Eine schematische Schnittansicht eines Teiles eines erfindungsgemäßen Raumluftreinigers gemäß einer weiteren Ausgestaltung der Erfindung;
Figur 7: Eine schematische Schnittansicht eines Teiles eines erfindungsgemäßen Raumluftreinigers gemäß einer weiteren Ausgestaltung der Erfindung;
Figur 8: Eine schematische Schnittansicht eines Teiles eines erfindungsgemäßen Raumluftreinigers gemäß einer weiteren Ausgestaltung der Erfindung;
Figur 9: Eine schematische Schnittansicht eines Teiles eines erfindungsgemäßen Raumluftreinigers gemäß einer weiteren Ausgestaltung der Erfindung;
Figur 10: Schematische Ansichten eines erfindungsgemäßen Raumluftreinigers gemäß einer weiteren Ausgestaltung der Erfindung, bei der die Stirnseite des Gehäuses, der die Luftaustrittsöffnung zugeordnet ist und die einen geringeren Durchmesser als das Gehäuse aufweist mittels eines Aktuators zwischen einer ersten und einer zweiten Position axial stufenlos verschiebbar ausgeführt ist;
Figur 11: Eine schematische Schnittansicht eines Teiles eines erfindungsgemäßen Raumluftreinigers gemäß einer weiteren Ausgestaltung der Erfindung; und
Figur 12: Eine schematische Schnittansicht einer weiteren Ausgestaltung eines erfindungsgemäßen Raumluftreinigers gemäß der Erfindung.

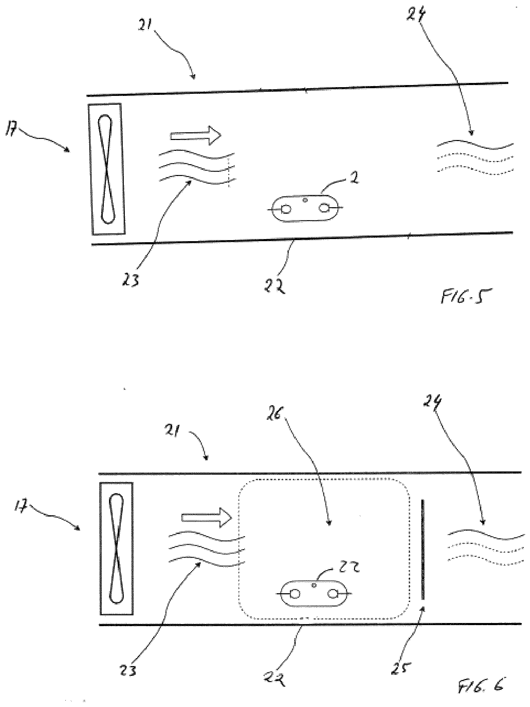

Gemäß der Erfindung und bezugnehmend auf Figur 5, welche eine schematische Schnittansicht eines Teiles eines erfindungsgemäßen Raumluftreinigers 21 gemäß einer Ausgestaltung der Erfindung darstellt, umfasst ein erfindungsgemäßer Raumluftreiniger 21 ein Gehäuse 22, in dem entlang einer Längsachse des Gehäuses 22, welche der Richtung seiner größten Ausdehnung entspricht oder achsparallel dazu ein Ozongenerator mit einer UV-Lampe 2 angeordnet ist. Ferner ist axial betrachtet an einer Stirnseite des Gehäuses 22 eine Lüftereinheit 17 angeordnet, welche eine Strömung von oder zu einer axial betrachtet gegenüberliegenden Stirnseite des Gehäuses 22 verursacht, wobei an beiden Stirnseiten des Gehäuses eine Öffnung vorgesehen ist, wobei in Abhängigkeit der durch die Lüftereinheit 17 verursachten Strömungsrichtung der Strömung eine Öffnung als Lufteintrittsöffnung und die andere Öffnung als Luftaustrittsöffnung ausgeführt ist, über die in das Geräteinnere angesaugte und mit Ozon angereicherte durchströmende Luft ausgestoßen wird.

Beim in Figur 5 gezeigten Beispiel erzeugt die Lüftereinheit 17 eine Strömung in Pfeilrichtung zu einer axial betrachtet gegenüberliegenden Stirnseite des Gehäuses 22. Die Luft 23 wird von der Lüftereinheit 17 über eine Lufteintrittsöffnung angesaugt und an der UV-Lampe 2 vorbeigeleitet, wobei sie zum Einen durch die UV-Strahlung entkeimt wird und zum Anderen mit Ozon angereichert wird, welches durch chemische Reaktionen mit Bestandteilen von Bakterien, Viren, Geruchs- und Schadstoffen diese größtenteils eliminiert. Die mit Ozon angereicherte Luft 24 wird über die Luftaustrittsöffnung ausgestoßen.

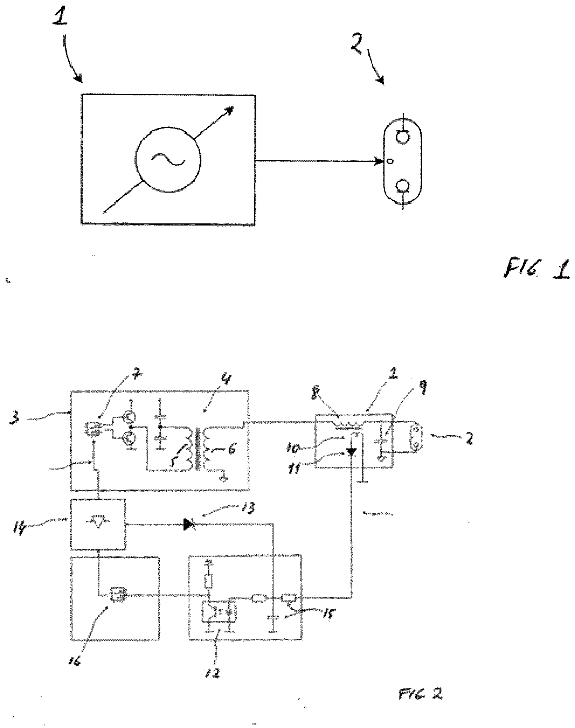

Bezugnehmend auf Figur 1 weist der Raumluftreiniger eine einstellbare Treiberschaltung 1 auf, welche die UV-Lampe 2 zündet und derart betreibt, dass die elektrische Leistung und somit die UV-Licht Strahlungsleistung einstellbar ist.

Bezugnehmend auf Figur 2 umfasst der Raumluftreiniger einen als Aufwärtswandler ausgeführten Spannungswandler 3 zur Erzeugung einer digital einstellbaren Spannung, dessen Ausgangsspannung als Eingangsspannung der einstellbaren Treiberschaltung 1 dient. Bei dem gezeigten Beispiel wird der Transformator 4, umfassend eine Primär- und eine Sekundärinduktivität 5, 6 durch einen integrierten Schaltkreis 7 gesteuert. Mittels des Transformators wird die Spannung an der Sekundärinduktivität 6 hochgesetzt. Hierbei ist ein digitales Potenziometer vorgesehen, welches eine Einstellung der Ausgangsspannung ermöglicht. Ein typischer Wert für die Spannungseinstellung ist eine Spannung zwischen 12 V und 24 V in 256 Schritten.

Die einstellbare Treiberschaltung 1 weist einen Resonanzkreis mit einer Resonanzinduktivität 8 und einen Resonanzkondensator 9 auf, wobei der Resonanzkreis durch die Ausgangsspannung des Spannungswandlers 3 in der Nähe seiner Resonanz angeregt wird, wodurch eine hohe Spannung entsteht, welche die Zündspannung der UV-Lampe 2 überschreitet. Bei intakter UV-Lampe wird diese gezündet, und der Resonanzkreis wird belastet. Hierbei wird dem Resonanzkreis Energie zum Betrieb der UV-Lampe 2 entnommen, wobei die UV-Lampe 2 mit einer Leistung leuchtet, die durch die Frequenz und die Eingangsspannung bestimmt ist. Höhere Eingangsspannung resultiert in einer höheren Leistung; ferner wird die Leistung erhöht, je näher sich die Frequenz an der Resonanzfrequenz des Resonanzkreises befindet.

Sollte die UV-Lampe 2 defekt sein, eine Unterbrechung in der Zuleitung auftreten, oder keine UV-Lampe 2 angeschlossen sein, bleibt der Resonanzkreis unbedämpft und die Zündspannung aufrecht. Diese Spannung wird in einer Sekundärwicklung 10 der Resonanzinduktivität 8 induziert, über eine Gleichrichterschaltung 11 einweggleichgerichtet, und über einen Optokoppler 12 erfasst, wobei, wenn die Spannung länger als eine vorgegebene Zeit bestehen bleibt, die Zehnerspannung einer einer Schutzschaltung 14 vorgeschalteten Zehnerdiode 13 überschritten wird, und Strom in die Schutzschaltung 14 fließt. Hierbei wird die Zeit bis zum Aktivieren der Schutzschaltung 14 durch einen Filter 15 bestimmt. Bei aktivierter Schutzschaltung wird der Leistungsteil angehalten, und die Leistungsübertragung stoppt. Die Schutzschaltung kann über einen Mikrocontroller 16 zurückgesetzt werden, um nach einer applizierbaren Abkühlzeit einen erneuten Zündversuch zu starten.

Der Mikrocontroller 16 ist mit dem Optokoppler 12 verbunden und erhält in Abhängigkeit der Spannung an der Sekundärwicklung10 der Resonanzinduktivität 8 ein Signal über den Status der UV-Lampe 2 (gezündet, nicht gezündet).

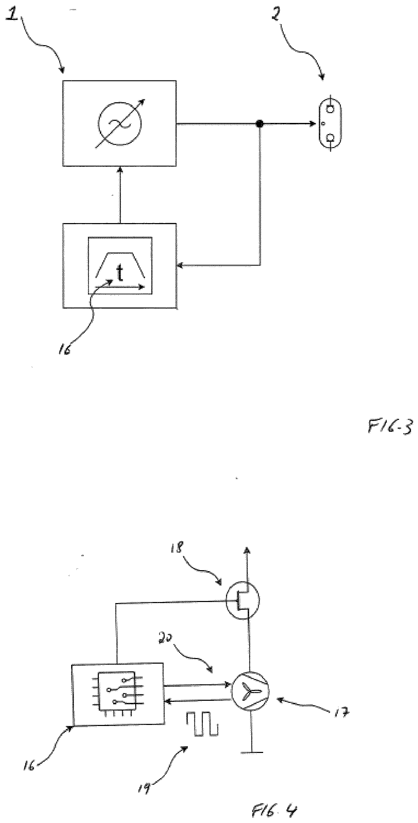

Auf diese Weise kann die Zeit, welche die UV-Lampe 2 zum Zünden benötigt über den Wechsel dieses Signals ermittelt werden, wie anhand Figur 3 veranschaulicht. Alterungseffekte in UV - Lampen bewirken, dass die Zeit bis zur erfolgreichen Zündung immer länger wird und von der Anzahl der Zündungen und der Brenndauer abhängt; sie erhöht sich so lange, bis die UV-Lampe unzuverlässig oder gar nicht mehr zündet und somit ihr Lebensdauernde erreicht hat und ausgetauscht werden muss. In vorteilhafter Weise ist es über die kontinuierliche Erfassung der Zeit, welche die UV-Lampe 2 zum Zünden benötigt, und eine entsprechende Auswertung im Mikrocontroller möglich, eine Lampenausfall - Früherkennung zu realisieren und einen bevorstehenden Lampenausfall zu erkennen.

Gemäß einer Weiterbildung der Erfindung kann mittels geeigneter Sensoren die Umgebungstemperatur und/oder die Lampentemperatur erfasst werden und bei der Auswertung zur Lampenausfall - Früherkennung zu berücksichtigen, da kalte Lampen eine längere Zeit zum Zünden benötigen. Alternativ oder zusätzlich kann der UV Output der UV-Lampe mittels zumindest eines UV-Sensors, der beispielsweise als Photodiode ausgeführt sein kann, direkt ermittelt werden. Da der UV-Output mit der Alterung der Lampe sinkt, kann auf diese eine frühzeitige Ausfallerkennung optimiert werden bei gleichzeitiger direkter Ermittlung eines Wirkungsverlustes der UV-Lampe.

Gemäß der Erfindung und bezugnehmend auf Figur 4 ist die Lüftereinheit 17 mittels des Mikrocontrollers 16 über eine Schaltung 18 aktivierbar und derart ausgeführt, dass die Drehzahl und somit der Luftdurchsatz bzw. die Auswurfstärke der Luft über Befehle 20 einstellbar ist. Ferner ist in die Lüftereinheit 17 ein mit dem Mikrocontroller verbundener Sensor integriert, welcher die Drehzahl der Lüftereinheit 17 erfasst und als Signal 19 an den Mikrocontroller zur Auswertung zum Zweck einer Lüftereinheitausfall - Früherkennung übermittelt. Durch Auswertung der Drehzahl der Lüftereinheit 17 kann erkannt werden, ob die Lüftereinheit 17 ihr Lebensdauerende erreicht hat, was beispielsweise bei einer durch defekte Lager ausgelösten Drehzahlreduzierung der Fall ist. Ferner kann erkannt werden, ob die Lüftereinheit 17 stillsteht.

Gegenstand der Figur 6 ist eine Weiterbildung der in Figur 5 gezeigten Ausführungsform. Hierbei weist der Raumluftreiniger 21 in Strömungsrichtung der Luft nach der UV-Lampe 2 ein fest angeordnetes Stauelement 25 vorgegebener Größe auf, durch das ein Rückstau der Luft 23 erzielt wird, was zu einer stärkeren Sättigung der Luft mit Ozon im Bestrahlungsraum 26 führt. Das Stauelement 25 ist senkrecht zur Luftstromrichtung angeordnet.

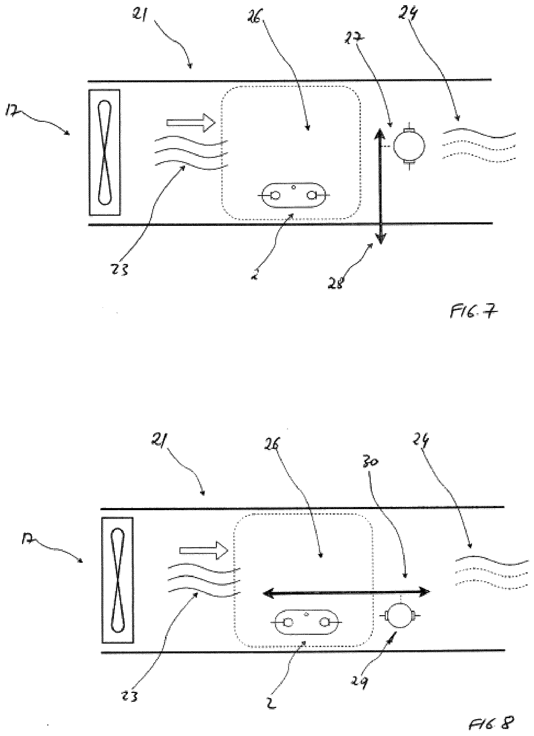

Im Rahmen einer Weiterbildung der in Figur 6 gezeigten Ausführungsform wird vorgeschlagen, dass der Raumluftreiniger in Strömungsrichtung der Luft nach dem UV-Lampe 2 ein mittels eines Aktuators 27 beweglich angeordnetes Stauelement 28 vorgegebener Größe aufweist, um einen regelbaren Rückstau der Luft 23 im Bestrahlungsraum 26 zu erzielen. Das Stauelement 28 ist senkrecht zur Luftstromrichtung in einer beliebigen Position zwischen einer Minimal- und einer Maximalposition anordenbar. In der Minimalposition wird kein Rückstau der Luft erzielt, wobei in der Maximalposition ein maximaler Wert für den Rückstau erzielt wird. Diese Ausgestaltung ist Gegenstand der Figur 7.

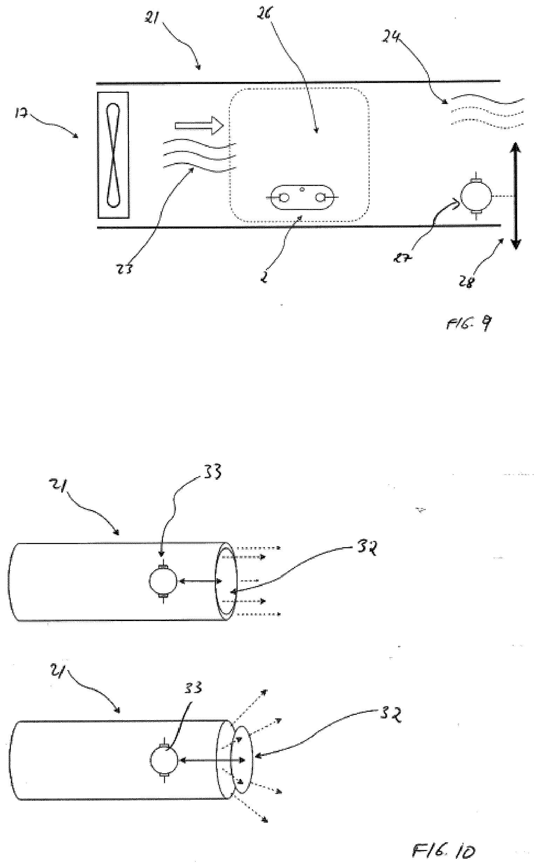

Das beweglich angeordnete Stauelement 28 kann, wie anhand Figur 9 veranschaulicht, nahe der Auslassöffnung vorgesehen sein, wodurch in vorteilhafter Weise über dessen Positionierung eine Veränderung des Ausstoßes, der Sättigung der Luft mit Ozon und des Volumenstroms erzielt wird.

Gemäß einer weiteren Ausgestaltung der Erfindung, welche mit den Ausgestaltungen gemäß Figuren 6 und 7 kombinierbar ist und bezugnehmend auf Figur 8, weist der Raumluftreiniger 21 ein mittels eines Aktuators 29 axial beweglich angeordnetes Stauelement 30 vorgegebener Größe auf, welches radial betrachtet in einem vorgegebenen Abstand zur UV-Lampe 2 anordenbar ist, derart, dass es zumindest einen Teil der UV-Lampe 2 abdeckt und den effektiven Bestrahlungsraum innerhalb des Raumluftreinigers 21 und somit den Ozonausstoß definiert verringert, da die Möglichkeit einer Interaktion der UV - Strahlung mit O2 auf den Bereich des Bestrahlungsraumes 26 zwischen der UV-Lampe 2 und dem Stauelement 30 eingeschränkt wird. Diese Ausgestaltung ist Gegenstand der Figur 7. Das Stauelement 30 ist zwischen einer Minimal- und einer Maximalposition anordenbar. In der Minimalposition wird die UV-Lampe 2 nicht abgedeckt, wobei in der Maximalposition eine vollständige Abdeckung der UV-Lampe 2 erzielbar ist.

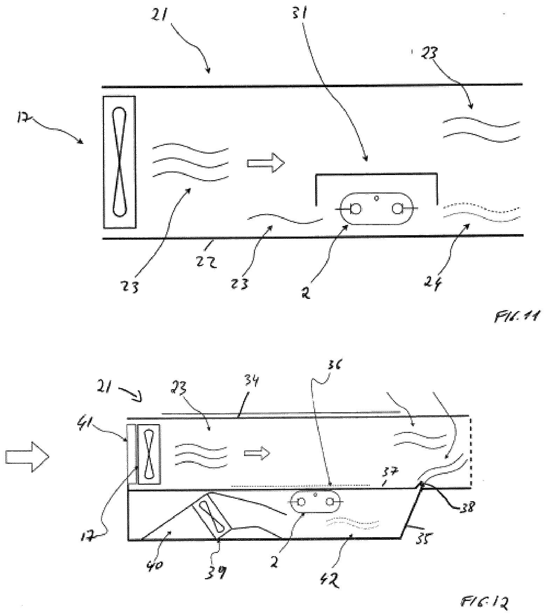

Ferner kann die UV-Lampe 2 radial und axial betrachtet von einem Luftleitbauteil 31 in einem vorgegebenen Abstand in einem vorgegebenen Winkelbereich umgeben sein, wobei zumindest die der UV-Lampe 2 zugewandte Seite des Luftleitbauteils für die vom Ozongenerator emittierte Strahlung reflektierend ausgeführt sein kann. Dadurch wird der Bestrahlungsraum innerhalb des Raumluftreinigers 21 und somit der Ozonausstoß verringert, da die Möglichkeit einer Interaktion der Luft 23 mit der UV - Strahlung der UV-Lampe 2 eingeschränkt wird. Diese Ausgestaltung ist Gegenstand der Figur 11 und kann mit den Ausgestaltungen gemäß Figuren 6, 7 und 8 kombiniert werden.

Gemäß einer Ausgestaltung der Erfindung weist die Stirnseite des Gehäuses, der die Luftaustrittsöffnung zugeordnet ist, bei einem zylinderförmigen Gehäuse einen geringeren Durchmesser auf als das Gehäuse und kann axial betrachtet um ein vorgegebenes Maß nach außen verschoben angeordnet sein. Für den Fall eines nicht zylinderförmigen Gehäuses ist die Fläche der Stirnseite kleiner, als die gedachte Fläche bei vollständig geschlossener Stirnseite des Gehäuses, wobei die Stirnseite axial betrachtet um ein vorgegebenes Maß nach außen verschoben angeordnet sein kann. Diese Ausgestaltungen sind mit den Ausgestaltungen nach Figuren 6, 7, 8 und 11 kombinierbar.

Wie in Figur 10 gezeigt, kann die Stirnseite 32 des Gehäuses 22, der die Luftaustrittsöffnung zugeordnet ist und die bei dem gezeigten Beispiel, bei dem das Gehäuse zylinderförmig ausgeführt ist, einen geringeren Durchmesser als das Gehäuse 22 aufweist, mittels eines Aktuators 33 zwischen einer ersten und einer zweiten Position axial stufenlos verschiebbar ausgeführt sein. In der ersten Position, die im oberen Teil der Figur 10 dargestellt ist, bei der die gedachte radiale Fortsetzung der Stirnseite 32 am Rand des Gehäuses 22 anliegt, wird die mit Ozon angereicherte Luft geradlinig und weit ausgestoßen, wobei in der zweiten Position, die im unteren Teil der Figur 10 dargestellt ist, bei der die Stirnseite 32 um einen Maximalwert nach außen verschoben angeordnet ist, die mit Ozon angereicherte Luft in den Raum in einem Winkelbereich zwischen 0 und 90° bezogen auf die Längsachse gleichmäßig konzentriert ausgestoßen wird. Bei einem nicht zylinderförmigen Gehäuse, bei dem die Fläche der Stirnseite kleiner ist, als die gedachte Fläche bei vollständig geschlossener Stirnseite des Gehäuses kann die Stirnseite analog zum beschriebenen Fall mittels eines Aktuators zwischen einer ersten und einer zweiten Position axial stufenlos verschiebbar ausgeführt sein. Diese Ausgestaltungen sind mit den Ausgestaltungen nach Figuren 6, 7, 8 und 11 kombinierbar.

Gemäß einer Weiterbildung der Erfindung kann in vorteilhafter Weise durch eine entsprechende Ansteuerung des Aktuators 33 ein zwischen der ersten und der zweiten Position oszillierender Luftausstoß realisiert werden.

Gemäß einer weiteren Ausgestaltung der Erfindung, die Gegenstand der Figur 12 ist, weist das Gehäuse 22 einen ersten Abschnitt 34 auf, welcher dem bereits beschriebenen Gehäuse entspricht, mit dem Unterschied, dass die UV-Lampe 2 nicht in diesem Gehäuseabschnitt, sondern in einem zweiten Gehäuseabschnitt 35 angeordnet ist, der vom ersten Gehäuseabschnitt 34 durch eine für die UV-A und UV-B Strahlung der UV-Lampe 2 über einen definierten Bereich 36 durchlässigen Wandabschnitt 37 aus beispielsweise Glas oder PMMA getrennt ist und durch eine in der Nähe der Luftauslassöffnung des ersten Gehäuseabschnitts 34, welche der Luftauslassöffnung des Raumluftreinigers 21 entspricht, vorgesehene variable Öffnung 38, welche als Einlassöffnung zum ersten Gehäuseabschnitt 34 dient, mit dem ersten Gehäuseabschnitt 34 strömungstechnisch verbunden ist. Vorzugsweise ist die variable Öffnung 38 als mittels eines Aktuators verstellbare Blende oder als elektronisch regelbare Düse ausgeführt.

Wie Figur 12 zu entnehmen ist, ist im zweiten Gehäuseabschnitt 35 eine weitere Lüftereinheit 39 vorgesehen, die über einen Anschluss 40 für Umluft, Außenluft oder gereinigte Raumluft einen an der UV-Lampe 2 vorbeiströmenden Luftstrom oder in Abhängigkeit der Anordnung der UV-Lampe 2 einen die UV-Lampe umströmenden Luftstrom in Richtung der variablen Öffnung 38 zum ersten Gehäuseabschnitt 34 generiert. Die weitere Lüftereinheit kann auch derart ausgeführt sein, dass die Lüfterdrehzahl einstellbar ist.

Erfindungsgemäß dient der erste Gehäuseabschnitt 34 als Reinigungskammer, in der die durch die Lüftereinheit 17 in das Geräteinnere angesaugte, z.B. durch Fett, Dreck, Staub verschmutzte Luft mit UV-A und UV-B Licht behandelt und gereinigt wird. Bei dem gezeigten Beispiel ist in Strömungsrichtung der Luft betrachtet der Lüftereinheit HEPA-Filterstufen 41 und ein nicht dargestellter Fettfilter aus Metall vorgeschaltet, die jedoch lediglich optional vorgesehen sein können.

Der zweite Gehäuseabschnitt 35 dient als Ozon-Generierungskammer, wobei die mit Ozon angereicherte Luft 42 der in dem als Reinigungskammer dienenden ersten Gehäuseabschnitt 34 gereinigten Luft in Strömungsrichtung vor der Luftauslassöffnung des ersten Gehäuseabschnitts 34 über die variable Öffnung 38 direkt regelbar beigemischt wird. Diese Ausgestaltung kann beispielsweise mit den Ausgestaltungen gemäß Figuren 8, 10 und 11 kombiniert werden. Ferner kann die Stirnseite des Gehäuses, der die Luftaustrittsöffnung zugeordnet ist, welche bei der Ausgestaltung nach Figur 12 die Stirnseite des ersten Gehäuseabschnitts ist, bei einem zylinderförmigen Gehäuse einen geringeren Durchmesser aufweisen als das Gehäuse und kann axial betrachtet um ein vorgegebenes Maß nach außen verschoben angeordnet sein. Für den Fall eines nicht zylinderförmigen Gehäuses ist die Fläche der Stirnseite kleiner, als die gedachte Fläche bei vollständig geschlossener Stirnseite des Gehäuses, wobei die Stirnseite axial betrachtet um ein vorgegebenes Maß nach außen verschoben angeordnet sein kann.

Gemäß einer Weiterbildung dieser Ausgestaltung kann die in der Ozon-Generierungskammer mit Ozon angereicherte Luft der in der Reinigungskammer gereinigten Luft in Strömungsrichtung vor der Luftauslassöffnung des ersten Gehäuseabschnitts in einer mit der Luftauslassöffnung des ersten Gehäuseabschnitts und mit der Reinigungskammer strömungstechnisch verbundenen Mischkammer über die variable Öffnung regelbar beigemischt werden. Hierbei werden die in der Reinigungskammer gereinigten Luft und die in der Ozon-Generierungskammer mit Ozon angereicherte Luft in die Mischkammer geleitet, welche mit der Luftauslassöffnung des ersten Gehäuseabschnitts zum Zweck des Ausstoßes des Gemisches strömungstechnisch verbunden ist. Der Ozonanteil der in der Ozon-Generierungskammer mit Ozon angereicherten Luft ergibt sich aus der eingestellten Leistung der UV-Lampe und der Drehzahl der in der Ozon-Generierungskammer angeordneten Lüftereinheit.

Ein erfindungsgemäßer Raumluftreiniger kann flexibel betrieben werden. Durch den Parameter "Leistung der UV-Lampe" kann die Ozongenerierung und somit die Anreicherung der angesaugten Luft mit Ozon beeinflusst werden; je höher die Leistung, desto höher ist der Ozonanteil. Die Ozongenerierung kann auch über den Parameter "Drehzahl der Lüftereinheit" beeinflusst werden; je niedriger die Drehzahl, desto höher ist der Ozonanteil, wobei die Auswurfstärke und somit die Reichweite der ausgestoßenen Luft mit sinkender Drehzahl abnimmt. Für Ausgestaltungen umfassend ein beweglich angeordnetes Stauelement können durch die Anordnung des Stauelementes die Ozongenerierung und die Auswurfstärke und somit die Reichweite der ausgestoßenen Luft ebenfalls beeinflusst werden; je höher der dem Luftstrom entgegengesetzter Widerstand, desto höher ist der Ozonanteil und desto geringer die Auswurfstärke und somit die Reichweite der ausgestoßenen Luft.

Bei der Ausgestaltung, wonach die Stirnseite des Gehäuses, der die Luftaustrittsöffnung zugeordnet ist, einen geringeren Durchmesser als das Gehäuse aufweist und mittels eines Aktuators zwischen einer ersten und einer zweiten Position axial stufenlos verschiebbar ausgeführt ist, kann über den Parameter "axiale Position der Stirnseite" bestimmt werden, ob die mit Ozon angereicherte Luft geradlinig und weit ausgestoßen wird oder ob die mit Ozon angereicherte Luft in den Raum in einem Winkelbereich zwischen 0 und 90° bezogen auf die Längsachse gleichmäßig konzentriert ausgestoßen wird.

## Patentansprüche

1. Raumluftreiniger (21) umfassend ein Gehäuse (22), in dem entlang einer Längsachse des Gehäuses (22), welche der Richtung seiner größten Ausdehnung entspricht oder achsparallel dazu ein Ozongenerator mit einer UV-Lampe (2) angeordnet ist, wobei axial betrachtet an einer Stirnseite des Gehäuses eine Lüftereinheit (17) angeordnet ist, welche eine Strömung von oder zu einer axial betrachtet gegenüberliegenden Stirnseite des Gehäuses (22) verursacht, wobei an beiden Stirnseiten des Gehäuses (22) eine Öffnung vorgesehen ist, wobei in Abhängigkeit der durch die Lüftereinheit (17) verursachten Strömungsrichtung der Strömung eine Öffnung als Lufteintrittsöffnung und die andere Öffnung als Luftaustrittsöffnung ausgeführt ist, über die in das Geräteinnere angesaugte und mit Ozon angereicherte durchströmende Luft ausgestoßen wird, wobei die Lüftereinheit (17) mittels des Mikrocontrollers (16) über eine Schaltung (18) aktivierbar und derart ausgeführt ist, dass die Drehzahl einstellbar ist, wobei in die Lüftereinheit (17) ein mit dem Mikrocontroller (16) verbundener Sensor integriert ist, welcher die Drehzahl der Lüftereinheit (17) erfasst und an den Mikrocontroller (16) zur Auswertung zum Zweck einer Lüftereinheitausfall - Früherkennung übermittelt, wobei der Raumluftreiniger eine einstellbare Treiberschaltung (1) aufweist, welche nach einem Signal eines Mikrocontrollers (16) die UV-Lampe (2) zündet und derart betreibt, dass die elektrische Leistung und somit die UV-Licht Strahlungsleistung einstellbar ist,
**dadurch gekennzeichnet, dass**, ein Übertrager vorgesehen ist, über den der Status der UV-Lampe (2) ausgekoppelt wird, wobei der Status der UV-Lampe, nämlich gezündet oder nicht gezündet, über einen Optokoppler (12) erfasst und an den Mikrocontroller (16) zur Auswertung zum Zweck einer Lampenausfall - Früherkennung übermittelt wird, wobei im Mikrocontroller (16) über den Wechsel dieses Signals die Zeit, welche die UV-Lampe (2) zum Zünden benötigt, ermittelbar ist und wobei über die kontinuierliche Erfassung der Zeit, welche die UV-Lampe (2) zum Zünden benötigt und anhand der Anzahl der Zündungen und der Brenndauer der UV-Lampe (2) im Mikrocontroller (16) eine Auswertung durchführbar ist, um einen bevorstehenden Lampenausfall zu erkennen.

2. Raumluftreiniger (21) nach Anspruch 1, **dadurch gekennzeichnet, dass** das Gehäuse (22) einen ersten Abschnitt (34) und einen zweiten Abschnitt (35) aufweist, in dem die UV-Lampe (2) angeordnet ist, wobei der zweite Abschnitt (35) vom ersten Abschnitt (34) durch eine für die UV-A und UV-B Strahlung der UV-Lampe (2) über einen definierten Bereich (36) durchlässigen Wandabschnitt (37) getrennt ist und durch eine in der Nähe der Luftauslassöffnung des ersten Abschnitts (34), welche der Luftauslassöffnung des Raumluftreinigers (21) entspricht, vorgesehene variable Öffnung (38), welche als Einlassöffnung zum ersten Abschnitt (34) dient, mit dem ersten Abschnitt (34) strömungstechnisch verbunden ist, wobei im zweiten Abschnitt (35) eine weitere Lüftereinheit (39) vorgesehen ist, welche über einen Anschluss (40) für Umluft, Außenluft oder gereinigte Raumluft einen an der UV-Lampe (2) vorbeiströmenden Luftstrom oder in Abhängigkeit der Anordnung der UV-Lampe (2) einen die UV-Lampe umströmenden Luftstrom in Richtung der variablen Öffnung (38) zum ersten Gehäuseabschnitt (34) generiert, wobei der erste Gehäuseabschnitt (34) als Reinigungskammer, in der die durch die Lüftereinheit (17) in das Geräteinnere angesaugte Luft mit UV-A und UV-B Licht behandelt und gereinigt wird, wobei der zweite Abschnitt (35) als dient Ozon-Generierungskammer dient, wobei die mit Ozon angereicherte Luft (42) der im ersten Abschnitt (34) gereinigten Luft in Strömungsrichtung vor der Luftauslassöffnung des ersten Abschnitts (34) über die variable Öffnung (38) direkt regelbar beigemischt wird.

3. Raumluftreiniger (21) nach Anspruch 1, **dadurch gekennzeichnet, dass** er in Strömungsrichtung der Luft nach der UV-Lampe (2) ein fest senkrecht zur Luftstromrichtung angeordnetes Stauelement (25) oder ein senkrecht zur Luftstromrichtung anordenbares, mittels eines Aktuators (27) beweglich angeordnetes Stauelement (28) vorgegebener Größe aufweist.

4. Raumluftreiniger (21) nach Anspruch 3, **dadurch gekennzeichnet, dass** das beweglich angeordnete Stauelement (28) nahe der Auslassöffnung vorgesehen ist, wodurch über dessen Positionierung eine Veränderung des Ausstoßes, der Sättigung der Luft mit Ozon und des Volumenstroms erzielbar ist.

5. Raumluftreiniger (21) nach Anspruch 1, 2, 3 oder 4, **dadurch gekennzeichnet, dass** er ein mittels eines Aktuators (29) axial beweglich angeordnetes Stauelement (30) vorgegebener Größe aufweist, welches radial betrachtet in einem vorgegebenen Abstand zur UV-Lampe (2) anordenbar ist, derart, dass es zumindest einen Teil der UV-Lampe (2) abdeckt und den effektiven Bestrahlungsraum innerhalb des Raumluftreinigers (21) definiert verringert.

6. Raumluftreiniger (21) nach Anspruch 1, 2, 3, 4 oder 5, **dadurch gekennzeichnet, dass** die UV-Lampe (2) radial und axial betrachtet von einem Luftleitbauteil (31) in einem vorgegebenen Abstand in einem vorgegebenen Winkelbereich umgeben ist.

7. Raumluftreiniger (21) nach Anspruch 1, 2, 3, 4, 5 oder 6, **dadurch gekennzeichnet, dass** die Stirnseite (32) des Gehäuses (22) bzw. des ersten Gehäuseabschnitts (34), der die Luftaustrittsöffnung zugeordnet ist, bei einem zylinderförmigen Gehäuse einen geringeren Durchmesser aufweist als das Gehäuse (22), wobei bei einem nicht zylinderförmigen Gehäuse die Fläche der Stirnseite kleiner ist, als die gedachte Fläche bei vollständig geschlossener Stirnseite des Gehäuses und wobei die Stirnseite (32) axial betrachtet um ein vorgegebenes Maß nach außen verschoben angeordnet ist.

8. Raumluftreiniger (21) nach Anspruch 1, 2, 3, 4, 5 oder 6, **dadurch gekennzeichnet, dass** die Stirnseite (32) des Gehäuses (22) bzw. des ersten Gehäuseabschnitts (34), der die Luftaustrittsöffnung zugeordnet ist und die bei einem zylinderförmigen Gehäuse (22), einen geringeren Durchmesser als das Gehäuse (22) aufweist und bei einem nicht zylinderförmigen Gehäuse eine Fläche aufweist, die kleiner ist, als die gedachte Fläche bei vollständig geschlossener Stirnseite, mittels eines Aktuators (33) zwischen einer ersten und einer zweiten Position axial stufenlos verschiebbar ausgeführt ist, wobei in der ersten Position die gedachte radiale Fortsetzung der Stirnseite (32) am Rand des Gehäuses (22) anliegt und in der zweiten Position die Stirnseite (32) um einen Maximalwert nach außen verschoben angeordnet ist.

9. Raumluftreiniger (21) nach einem der vorangehenden Ansprüche, **dadurch gekennzeichnet, dass** er zumindest einen Sensor aufweist oder mit zumindest einem Sensor über einen geeigneten Standard zur drahtlosen Kommunikation, verbindbar ist, wobei der zumindest eine Sensor vom Raumluftreiniger (21) periodisch abgefragt wird oder periodisch Sensordaten an den Raumluftreiniger (21) übermittelt, welcher anhand der Sensordaten steuerbar ist.

10. Raumluftreiniger (21) nach Anspruch 9, **dadurch gekennzeichnet, dass** der zumindest eine Sensor als Anwesenheitssensor, Mikrofon, als Sensor zur Erfassung der Ozonkonzentration oder als Luftqualitätssensor ausgeführt ist.

11. Verfahren zum Betreiben eines nach einem der Ansprüche 1-10 ausgeführten Raumluftreinigers (21), **dadurch gekennzeichnet, dass** eine Lampenausfall - Früherkennung durchgeführt wird, wobei der Mikrocontroller (16) über den Optokoppler (12) ein Signal über den Status der UV-Lampe (2), nämlich gezündet oder nicht gezündet, erhält, wobei über den Wechsel dieses Signals die Zeit, welche die UV-Lampe (2) zum Zünden benötigt ermittelt wird, wobei über die kontinuierliche Erfassung der Zeit, welche die UV-Lampe (2) zum Zünden benötigt und anhand der Anzahl der Zündungen und der Brenndauer der UV-Lampe (2) im Mikrocontroller (16) eine Auswertung durchgeführt wird, um einen bevorstehenden Lampenausfall zu erkennen.

12. Verfahren zum Betreiben eines Raumluftreinigers (21), nach Anspruch 11, **dadurch gekennzeichnet, dass** mittels geeigneter Sensoren die Umgebungstemperatur und/oder die Temperatur der UV-Lampe (2) erfasst werden und bei der Auswertung zur Lampenausfall - Früherkennung berücksichtigt werden und/oder dass der UV-Output der UV-Lampe (2), welcher mit der Alterung der UV-Lampe (2) sinkt, mittels zumindest eines UV-Sensors direkt ermittelt wird und bei der Auswertung zur Lampenausfall - Früherkennung berücksichtigt wird und/oder dass ein Sensor zur Erfassung der Ozonkonzentration verwendet wird, um den Zustand der UV-Lampe (2) zu überprüfen, wobei die Ozonkonzentration nach dem Einschalten des Gerätes kontinuierlich erfasst wird und wobei, wenn diese nicht wie erwartet in Abhängigkeit der Raumgröße und des Abstandes des Sensors zur Erfassung der Ozonkonzentration zum Gerät steigt, ein Defekt der UV-Lampe (2) erkannt wird.

13. Verfahren zum Betreiben eines Raumluftreinigers (21), nach Anspruch 11 oder 12, **dadurch gekennzeichnet, dass** im Rahmen der Lüftereinheitausfall - Früherkennung ein bevorstehender Lüftereinheitausfall erkannt wird, wenn eine niedrigere Drehzahl als nominal erwartet erfasst wird.

14. Verfahren zum Betreiben eines Raumluftreinigers (21), nach Anspruch 11, 12 oder 13, **dadurch gekennzeichnet, dass** Raumluftreiniger (21) einer Gruppe zugeordnet werden, wobei beliebige Geräte zu einer Gruppe zugeordnet oder herausgenommen werden können, wobei sämtliche derselben Gruppe zugeordneten Geräte synchronisiert oder definiert zu Bedienereingaben an einem der dieser Gruppe zugeordneten Gerät reagieren, wobei die Art der Bedienereingabe lokal, über ein Netzwerk und ein Modul zur drahtlosen Kommunikation oder über eine Fernbedienung erfolgen kann und wobei Raumluftreiniger einer Gruppe miteinander kommunizieren.

15. Verfahren zum Betreiben eines Raumluftreinigers (21), nach Anspruch 14, **dadurch gekennzeichnet, dass** die synchrone Steuerung der Raumluftreiniger (21) einer Gruppe dadurch erfolgt, dass in den Raumluftreinigern (21) jeder Gruppe eine eindeutige Kennung sämtlicher Raumluftreiniger (21) der Gruppe gespeichert wird, so dass, wenn ein Raumluftreiniger (21) dieser Gruppe eine Bedienereingabe empfängt, diese ausführt und adressiert an alle Raumluftreiniger (21) in derselben Gruppe weiter sendet oder dass ein Raumluftreiniger (21) einer Gruppe nach Erhalt einer Bedienereingabe diese ausführt und einen Broadcast - Befehl an alle Raumluftreiniger (21) in allen Gruppen sendet, welcher an die Raumluftreiniger (21) der Gruppe, der der Raumluftreiniger (21) zugeordnet ist, adressiert ist, wobei aufgrund der in jedem Raumluftreiniger (21) gespeicherten Informationen über die Gruppenzugehörigkeit die Bedienereingabe nur von den Raumluftreinigern (21) der Gruppe ausgeführt wird, der der broadcastende Raumluftreiniger (21) zugeordnet ist oder dass die Raumluftreiniger (21) einer Gruppe gleichzeitig über ein Netzwerk und die Module zur drahtlosen Kommunikation der Geräte gesteuert werden.

16. Verfahren zum Betreiben eines Raumluftreinigers (21), nach Anspruch 14, **dadurch gekennzeichnet, dass** Raumluftreiniger (21) einer Gruppe, die sich am selben Ort befinden, durch einen der Raumluftreiniger (21) anhand von Signalen eines diesem Raumluftreiniger (21) zugeordneten Luftqualitätssensors gesteuert werden, wobei, wenn mittels des Luftqualitätssensors detektiert wird, dass die Luftqualität einen Schwellwert unterschritten hat, der Raumluftreiniger, dem der Luftqualitätssensor zugeordnet ist aktiviert wird, wobei die weiteren Raumluftreiniger (21) der Gruppe sofort oder nach Ablauf eines vorgegebenen, einstellbaren Timers, wenn bis zum Ablauf des Timers die Luftqualität nicht in einem vorgegebenen Maße verbessert ist, aktiviert werden und wobei anschließend alle Raumluftreiniger (21) deaktiviert werden, wenn die Luftqualität den Schwellenwert nicht mehr unterschreitet oder die Luftqualität einen weiteren Schwellenwert überschreitet, der höher als der Schwellenwert ist, welcher die Aktivierung verursacht hat.

## Claims

1. A room air purifier (21) comprising a housing (22), in which an ozone generator with a UV lamp (2) is arranged along a longitudinal axis of the housing (22), which corresponds to the direction of its largest extension, or runs axis-parallel thereto, wherein a ventilator unit (17) when viewed axially is arranged at an end face of the housing, which causes a flow from or to an opposite end face of the housing (22) as viewed axially, wherein an opening is provided at both end faces of the housing (22), wherein, depending on the flow direction of the flow caused by the ventilator unit (17) one opening is constituted as an air inlet opening and the other opening as an air outlet opening, via which through-flowing air sucked into the device interior and enriched with ozone is expelled, wherein the ventilator unit (17) can be activated by means of the microcontroller (16) via a switch (18) and is constituted such that the speed can be adjusted, wherein a sensor connected to the microcontroller (16) is integrated into the ventilator unit (17), which detects the speed of the ventilator unit (17) and transmits it to the microcontroller (16) for evaluation for the purpose of a ventilator unit failure - early detection, wherein the room air purifier comprises an adjustable driver circuit (1), which ignites the UV lamp (2) after a signal of a microcontroller (16) and operates in such a way that the electrical power and thus the UV light radiation power can be adjusted,
**characterised in that** a transmitter is provided, via which the status of the UV lamp (2) is decoupled, wherein the status of the UV lamp, i.e. ignited or not ignited, is detected via an optocoupler (12) and transmitted to the microcontroller (16) for evaluation for the purpose of a lamp failure - early recognition, wherein the time required to ignites the UV lamp (2) can be determined in the microcontroller (16) by the change of this signal, and wherein an evaluation can be carried out in the microcontroller (16) by the continuous detection of the time which is required to ignite the UV lamp (2) and on the basis of the number of ignitions and the burning time of the UV lamp (2) in order to detect an imminent lamp failure.

2. The room air purifier (21) according to claim 1, **characterised in that** the housing (22) comprises a first section (34) and a second section (35), in which the UV lamp (2) is arranged, wherein the second section (35) is separated from the first section (34) by a wall section (37) permeable for UV-A and UV-B radiation of the UV lamp (2) over a defined range (36) and is connected in a flow-related manner to the first section (34) by a variable opening (38), which serves as an inlet opening to the first section (34), provided in the vicinity of the air outlet opening of the first section (34), which corresponds to the air outlet opening of the room air purifier (21), wherein a further ventilator unit (39) is provided in the second section (35), which generates, via a connection (40) for surrounding air, external or purified room air, an air flow flowing past the UV lamp (2) or, depending on the arrangement of the UV lamp (2), an air flow flowing around the UV lamp in the direction of the variable opening (38) to the first housing section (34), wherein the first housing section (34) as a purifying chamber, in which the air sucked into the device interior by the ventilator unit (17) is treated and purified with UV-A and UV-B light, wherein the second section (35) serves as an ozone generating chamber, wherein the air (42) enriched with the ozone is directly mixed in a regulated manner with the air purified in the first section (34) in the flow direction before the air outlet opening of the first section (34) via the variable opening (38).

3. The room air purifier (21) according to claim 1, **characterised in that** it comprises in the flow direction of the air after the UV lamp (2) a baffle element (25) arranged fixedly at right angles to the air flow direction or a baffle element (28) of predefined size arranged mobile by means of an actuator (27) which can be arranged at right angles to the flow direction.

4. The room air purifier (21) according to claim 3, **characterised in that** the baffle element (28) arranged to mobile is provided close to the outlet opening, wherein a change in the output, the saturation of the air with ozone and the volume flow can be achieved by its positioning.

5. The room air purifier (21) according to claim 1, 2, 3 or 4, **characterised in that** it comprises a baffle element (30) of predefined size arranged axially mobile by means of an actuator (29), which can be arranged when viewed radially at a predefined distance from UV lamp (2), in such a way that it covers at least a part of the UV lamp (2) and reduces the effective radiation space inside the room air purifier (21) in a defined manner.

6. The room air purifier (21) according to claim 1, 2, 3, 4 or 5, **characterised in that** the UV lamp (2) when viewed radially and axially is surrounded by an airdirecting component (31) at a predefined distance in a predefined angular range.

7. The room air purifier (21) according to claim 1, 2, 3, 4, 5 or 6, **characterised in that** the end face (32) of the housing (22) or of the first housing section (34) to which the air outlet opening is assigned, in the case of a cylindrical housing has a smaller diameter than the housing (22), wherein in the case of a non-cylindrical housing the area of the end face is smaller than the imaginary area in the case of a completely closed end face of the housing and wherein the end face (32) when viewed axially is arranged displaced outwards by a predefined amount.

8. The room air purifier (21) according to claim 1, 2, 3, 4, 5 or 6, **characterised in that** the end face (32) of the housing (22) or the first housing section (34), to which the air outlet opening is assigned and which in the case of a cylindrical housing (22) has a smaller diameter than the housing (22) and in the case of a non-cylindrical housing has an area which is smaller than the imaginary area with a completely closed end face, is constituted axially displaceable in a stepless manner by means of an actuator (33) between a first and a second position, wherein in the first position the imaginary radial continuation of the end face (32) lies at the edge of the housing (22) and in the second position the end face (32) is arranged displaced outwards by a maximum amount.

9. The room air purifier (21) according to any one of the preceding claims, **characterised in that** it comprises at least one sensor and can be connected to the at least one sensor via a suitable standard for wireless communication, wherein the at least one sensor is periodically interrogated by the room air purifier (21) or periodically transmits sensor data to the room air purifier (21), which can be controlled with the aid of sensor data.

10. The room air purifier (21) according to claim 9, **characterised in that** the at least one sensor is designed as a presence sensor, a microphone, as a sensor for detecting the ozone concentration or as an air quality sensor.

11. A method for the operation of a room air purifier (21) constituted according to any one of claims 1-10, **characterised in that** a lamp failure - early detection is carried out, wherein the microcontroller (16) receives a signal via the optocoupler (12) concerning the status of the UV lamp (2), i.e. ignited or not ignited, wherein the time which the UV lamp (2) requires for ignition is determined via the change in this signal, wherein an evaluation is carried out in the microprocessor (16) via the continuous detection of the time which the UV lamp (2) requires for ignition and on the basis of the number of ignitions and the burning time of the UV lamp (2), in order to detect an imminent lamp failure.

12. The method for operating a room air purifier (21) according to claim 11, **characterised in that** the ambient temperature and/or the temperature of the UV lamp (2) are detected by means of suitable sensors and are taken into account in the evaluation of the lamp failure - early detection and/or that the UV output of the UV lamp (2), which falls with the ageing of the UV lamp (2), is determined directly by means of at least one UV sensor and is taken into account in the evaluation of the lamp failure - early detection, and/or that a sensor for the detection of the ozone concentration is used to check the state of the UV lamp (2), wherein the ozone concentration is continuously detected after the device is switched on and wherein, when this does not increase as expected depending on the room size and the distance of the sensor for the detection of the ozone concentration from the device, a defect of the UV lamp (2) is detected.

13. The method for operating a room air purifier (21) according to claim 11 or 12, **characterised in that**, in the context of the ventilator unit failure - early detection, an imminent ventilator unit failure is detected if a lower speed than nominally expected is detected.

14. The method for operating a room air purifier (21) according to claim 11, 12 or 13, **characterised in that** the room air purifiers (21) are assigned to a group, wherein any devices can be assigned to or removed from a group, wherein all of the devices assigned to the same group react in a synchronised and defined manner to user inputs to a device assigned to this group, wherein the type of user input can take place locally, via a network and a module for wireless communication or via remote control and wherein room air purifiers of a group communicate with one another.

15. The method for operating a room air purifier (21) according to claim 14, **characterised in that** the synchronous control of the room air purifiers (21) of a group takes place by the fact that in the room air purifiers (21) of each group an unique identifier of all the room air purifiers (21) of the group is stored, in such a way that, when a room air purifier (21) of this group receives an operator input, executes the latter and relays it addressed to all the room air purifiers (21) in the same group, or that a room air purifier (21) of a group, after receiving an operator input, executes the latter and sends a broadcast - command to all the room air purifiers (21) in all groups, which is addressed to the room air purifiers (21) of the group to which the room air purifier (21) is assigned, wherein, due to the information on their belonging to the group which is stored in each room air purifier (21), the operator input is executed only by the room air purifiers (21) of the group to which the broadcasting room air purifier (21) is assigned or that the room air purifiers (21) of a group are simultaneously controlled via a network and the modules for wireless communication of the devices.

16. The method for operating a room air purifier (21) according to claim 14, **characterised in that** room air purifiers (21) of a group, which are located in the same place, are controlled one of the room air purifiers (21) with the aid of signals of an air quality sensor assigned to this room air purifier (21), wherein, when it is detected by the air quality sensor that the air quality has fallen below a threshold value, the room air purifier, to which the air quality sensor is assigned, is activated, wherein the further room air purifiers (21) of the group immediately or after the lapse of a predefined, predeterminable timer, if up to the lapse of the timer the air quality has not improved to a predefined extent, are activated and wherein all the room air purifiers (21) are then deactivated, if the air quality no longer falls below the threshold value or the air quality exceeds a further threshold value, which is higher than the threshold which has caused the activation.

## Revendications

1. Dispositif de nettoyage de l'air ambiant (21) comprenant un boîtier (22), dans lequel le long d'un axe longitudinal du boîtier (22), lequel correspond à la direction de son extension maximale ou en plus parallèlement à l'axe, est disposé un générateur d'ozone doté d'une lampe UV (2), sachant que, vue dans le sens axial, une unité de ventilation (17) est disposée sur une face avant du boîtier, laquelle cause un écoulement depuis ou vers une face avant du boîtier (22) opposée, vue dans le sens axial, sachant que sur les deux faces avant du boîtier (22), une ouverture est prévue, sachant qu'en fonction de la direction d'écoulement de l'écoulement, causée par l'unité de ventilation (17), une ouverture est réalisée en tant qu'ouverture d'entrée d'air et l'autre ouverture en tant qu'ouverture de sortie d'air par le biais de laquelle l'air aspiré à l'intérieur de l'appareil et s'écoulant à travers enrichi à l'ozone est rejeté, sachant que l'unité de ventilation (17) peut être activée au moyen du microcontrôleur (16) par le biais d'un circuit (18) et est exécutée de telle manière que la vitesse de rotation peut être réglée, sachant que dans l'unité de ventilation (17) est intégré un capteur relié au microcontrôleur (16), lequel saisit la vitesse de rotation de l'unité de ventilation (17) et la transmet au microcontrôleur (16) pour évaluation dans le but d'une identification précoce de panne de l'unité de ventilation, sachant que le dispositif de nettoyage de l'air ambiant comporte un circuit pilote (1) réglable, lequel allume la lampe UV (2) d'après un signal d'un microcontrôleur (16) et la fait fonctionner de telle manière que la puissance électrique et de ce fait la puissance de rayonnement de la lumière UV peuvent être réglées,
**caractérisé en ce qu'**un transformateur est prévu par le biais duquel l'état de la lampe UV (2) est découplé, sachant que l'état de la lampe UV, à savoir allumé ou non allumé, est saisi par un optocoupleur (12) et est transmis au microcontrôleur (16) pour évaluation dans le but d'une identification précoce de panne de lampe, sachant que dans le microcontrôleur (16), le temps nécessaire à la lampe UV (2) pour s'allumer, peut être déterminé par le changement de ce signal et sachant que par la saisie continue du temps qui est nécessaire à la lampe UV (2) pour s'allumer et à l'aide du nombre d'allumages et de la durée d'éclairage de la lampe UV (2), une évaluation peut être effectuée dans le microcontrôleur (16) pour identifier une panne de lampe imminente.

2. Dispositif de nettoyage de l'air ambiant (21) selon la revendication 1, **caractérisé en ce que** le boîtier (22) comporte une première section (34) et une deuxième section (35) dans lequel est disposée la lampe UV (2), sachant que la deuxième section (35) est séparée de la première section (34) par une section de paroi (37) perméable au rayonnement UV-A et UV-B de la lampe UV (2) sur une zone définie (36) et est reliée fluidiquement à la première section (34) par une ouverture (38) variable prévue à proximité de l'ouverture de sortie d'air de la première section (34), laquelle correspond à l'ouverture de sortie d'air du dispositif de nettoyage de l'air ambiant (21), laquelle sert d'ouverture d'entrée à la première section (34)sachant que dans la deuxième section (35), une autre unité de ventilation (39) est prévue, laquelle génère par le biais d'un raccord (40) pour air de circulation, air extérieur ou air ambiant purifié, un écoulement d'air passant sur la lampe UV (2) ou en fonction de la disposition de la lampe UV (2), un écoulement d'air s'écoulant autour de la lampe UV (2) en direction de l'ouverture variable (38) vers la première section de boîtier (34), sachant que la première section de boîtier (34) sert de chambre de purification, dans laquelle l'air aspiré par l'unité de ventilation (17) à l'intérieur de l'appareil est traité et purifié avec de la lumière UV-A et UV-B, sachant que la deuxième section (35) sert de chambre de génération d'ozone, sachant que l'air enrichi à l'ozone (42) est ajouté à l'air purifié dans la première section (34) de façon directement réglable par l'ouverture variable (38) en direction de l'écoulement avant l'ouverture de sortie d'air de la première section (34).

3. Dispositif de nettoyage de l'air ambiant (21) selon la revendication 1, **caractérisé en ce qu'**il comporte en direction d'écoulement de l'air après la lampe UV (2), un élément de retenue (25) disposé fixe perpendiculairement à la direction d'écoulement d'air ou un élément de retenue (28) de taille prédéfinie, pouvant être disposé perpendiculairement à la direction d'écoulement d'air, disposé de façon mobile au moyen d'un actionneur (27).

4. Dispositif de nettoyage de l'air ambiant (21) selon la revendication 3, **caractérisé en ce que** l'élément de retenue (28) disposé de façon mobile est prévu près de l'ouverture de sortie par le positionnement duquel il est possible d'obtenir une modification du rejet, de la saturation de l'air avec l'ozone et du débit.

5. Dispositif de nettoyage de l'air ambiant (21) selon la revendication 1, 2, 3 ou 4, **caractérisé en ce qu'il** comporte un élément de retenue (30) d'une taille prédéfinie, disposé axialement mobile au moyen d'un actionneur (29), lequel, vu dans le sens radial, peut être disposé à une distance prédéfinie de la lampe UV (2) de telle manière qu'il couvre au moins une partie de la lampe UV (2) et réduit de façon définie l'espace de rayonnement effectif à l'intérieur du dispositif de nettoyage de l'air ambiant (21).

6. Dispositif de nettoyage de l'air ambiant (21) selon la revendication 1, 2, 3, 4 ou 5, **caractérisé en ce que** la lampe UV (2) est entourée, vue dans le sens radial et axial, par un composant déflecteur d'air (31) à une distance prédéfinie dans une zone angulaire prédéfinie.

7. Dispositif de nettoyage de l'air ambiant (21) selon la revendication 1, 2, 3, 4, 5 ou 6, **caractérisé en ce que** la face avant (32) du boîtier (22) ou de la première section de boîtier (34), à laquelle l'ouverture de sortie d'air est attribuée, comporte pour un boîtier cylindrique, un diamètre plus faible que le boîtier (22), sachant que pour un boîtier non cylindrique, la surface de la face avant est plus petite que la surface considérée pour la face avant complètement fermée du boîtier et sachant que la face avant (32), vue dans le sens axial, est disposée déplacée vers l'extérieur d'une mesure prédéfinie.

8. Dispositif de nettoyage de l'air ambiant (21) selon la revendication 1, 2, 3, 4, 5 ou 6, **caractérisé en ce que** la face avant (32) du boîtier (22) ou de la première section de boîtier (34), à laquelle est attribuée l'ouverture de sortie d'air et qui comporte, pour un boîtier cylindrique (22), un diamètre plus faible que le boîtier (22) et comporte pour un boîtier non cylindrique, une surface, qui est plus petite que la surface considérée pour la face avant complètement fermée, est réalisée pouvant être déplacée axialement en continu au moyen d'un actionneur (33) entre une première et une deuxième position, sachant que dans la première position, le prolongement radial considéré de la face avant (32) s'applique au bord du boîtier (22) et dans la deuxième position, la face avant (32) est disposée déplacée vers l'extérieur d'une valeur maximale.

9. Dispositif de nettoyage de l'air ambiant (21) selon l'une quelconque des revendications précédentes, **caractérisé en ce qu'**il comporte au moins un capteur ou peut être relié à au moins un capteur par le biais d'une norme appropriée de communication sans fil, sachant qu'au moins un capteur est périodiquement interrogé par le dispositif de nettoyage de l'air ambiant (21) ou transmet périodiquement les données de détection au dispositif de nettoyage de l'air ambiant (21), lequel peut être piloté à l'aide de données de détection.

10. Dispositif de nettoyage de l'air ambiant (21) selon la revendication 9, **caractérisé en ce qu'**au moins un capteur est réalisé sous la forme d'un capteur de présence, d'un microphone, sous la forme d'un capteur de saisie de la concentration d'ozone ou sous la forme d'un capteur de la qualité de l'air.

11. Procédé de fonctionnement d'un dispositif de nettoyage de l'air ambiant (21) réalisé selon l'une quelconque des revendications 1-10, **caractérisé en ce qu'**une identification précoce de panne de lampe est effectuée, sachant que le microcontrôleur (16) reçoit par le biais de l'optocoupleur (12) un signal relatif à l'état de la lampe UV (2), à savoir allumé ou non allumé, sachant que par le biais du changement de ce signal, le temps qui est nécessaire à la lampe UV (2) pour s'allumer est déterminé, sachant que par la saisie continue du temps qui est nécessaire à la lampe UV (2) pour s'allumer et à l'aide du nombre d'allumages et de la durée d'éclairage de la lampe UV (2) une évaluation est effectuée dans le microcontrôleur (16) pour identifier une panne de lampe imminente.

12. Procédé de fonctionnement d'un dispositif de nettoyage de l'air ambiant (21) selon la revendication 11, **caractérisé en ce qu'**au moyen de capteurs appropriés, la température ambiante et/ou la température de la lampe UV (2) sont saisies et sont prises en considération lors de l'évaluation pour l'identification précoce de la panne de lampe et/ou **en ce que** la puissance UV de la lampe UV (2), laquelle décroit avec le vieillissement de la lampe UV (2), est déterminée directement au moyen d'au moins un capteur d'UV et est prise en considération lors de l'évaluation de l'identification précoce de la panne de lampe et/ou **en ce qu'**un capteur est utilisé pour la saisie de la concentration d'ozone pour vérifier l'état de la lampe UV (2), sachant que la concentration d'ozone est saisie en continu après la mise en marche de l'appareil et sachant que, si celle-ci n'augmente pas comme attendu en fonction de la taille du local et de la distance du capteur pour la saisie de la concentration d'ozone par rapport à l'appareil, un défaut de la lampe UV (2) est identifié.

13. Procédé de fonctionnement d'un dispositif de nettoyage de l'air ambiant (21) selon la revendication 11 ou 12, **caractérisé en ce que** dans le cadre de l'identification précoce de la panne de l'unité de ventilation, une panne de l'unité de ventilation imminente est identifiée, lorsqu'une vitesse de rotation plus faible que la vitesse nominale attendue, est saisie.

14. Procédé de fonctionnement d'un dispositif de nettoyage de l'air ambiant (21) selon la revendication 11, 12 ou 13, **caractérisé en ce que** des dispositifs de nettoyage de l'air ambiant (21) sont attribués à un groupe, sachant que n'importe quels appareils peuvent être attribués à un groupe ou enlevés, sachant que tous les appareils attribués au même groupe réagissent de façon synchronisée ou définie aux instructions d'opérateur sur un appareil attribué à ce groupe, sachant que le type d'instruction d'opérateur peut avoir lieu localement par le biais d'un réseau et d'un module de communication sans fil ou par le biais d'une télécommande et sachant que les dispositifs de nettoyage de l'air ambiant d'un groupe communiquent entre eux.

15. Procédé de fonctionnement d'un dispositif de nettoyage de l'air ambiant (21) selon la revendication 14 **caractérisé en ce que** la commande synchrone des dispositifs de nettoyage de l'air ambiant (21) d'un groupe a lieu par le fait que dans les dispositifs de nettoyage de l'air ambiant (21) de chaque groupe, une caractéristique incontestable de tous les dispositifs de nettoyage de l'air ambiant (21) du groupe est mémorisée de telle manière que lorsqu'un dispositif de nettoyage de l'air ambiant (21) de ce groupe reçoit une instruction d'opérateur, il exécute et adresse celle-ci et la retransmet à tous les dispositifs de nettoyage de l'air ambiant (21) dans le même groupe ou **en ce qu'**un dispositif de nettoyage de l'air ambiant (21) d'un groupe exécute celle-ci après réception d'une instruction d'opérateur et émet un ordre de diffusion à tous les dispositifs de nettoyage de l'air ambiant (21) dans tous les groupes, lequel est adressé aux dispositifs de nettoyage de l'air ambiant (21) du groupe auquel le dispositif de nettoyage de l'air ambiant (21) est attribué, sachant qu'en raison des informations mémorisées dans chaque dispositif de nettoyage de l'air ambiant (21) relatives à l'appartenance au groupe, l'instruction d'opérateur n'est exécutée que par les dispositifs de nettoyage de l'air ambiant (21) du groupe, auquel le dispositif de nettoyage de l'air ambiant (21) diffusant est attribué ou **en ce que** les dispositifs de nettoyage de l'air ambiant (21) d'un groupe sont simultanément pilotés par le biais d'un réseau et de modules de communication sans fil des appareils.

16. Procédé de fonctionnement d'un dispositif de nettoyage de l'air ambiant (21) selon la revendication 14, **caractérisé en ce que** des dispositifs de nettoyage de l'air ambiant (21) d'un groupe, qui se trouvent au même endroit, sont commandés par un des dispositifs de nettoyage de l'air ambiant (21) à l'aide de signaux d'un capteur de qualité de l'air attribué à ce dispositif de nettoyage de l'air ambiant (21), sachant que, si à l'aide du capteur de qualité de l'air il est détecté que la qualité de l'air est tombée en dessous d'une valeur seuil, le dispositif de nettoyage de l'air ambiant auquel le capteur de qualité de l'air est attribué, est activé, sachant que les autres dispositifs de nettoyage de l'air ambiant (21) du groupe sont activés immédiatement ou après expiration de la durée d'une minuterie réglable, prédéfinie, si la qualité de l'air n'est pas améliorée dans une mesure prédéfinie avant l'expiration de la durée de la minuterie et sachant ensuite que tous les dispositif de nettoyage de l'air ambiant (21) sont désactivés, si la qualité de l'air ne tombe plus sous la valeur seuil ou la qualité de l'air dépasse une autre valeur seuil, qui est supérieure à la valeur seuil, laquelle a causé l'activation.
